# EUROPEAN PATENT APPLICATION

(11) **EP 2 665 008 A1**
(43) Date of publication of application: **20.11.2013**
(21) Application number: 13151900.1
(22) Date of filing: 18.01.2013
(51) Int. Cl.: G06F 19/00

(54) **Exercise metric graphical code generation**

(30) Priority: 18.05.2012 US 201213475660
(71) Applicant: Precor Incorporated, Woodinville, WA 98072 (US)
(72) Inventor: Zuber, Nathan S., Woodinville, WA Washington 98072-4002 (US)
(74) Representative: Docherty, Andrew John

(57) **Abstract**

Different graphical codes are generated and displayed based upon signals representing different values of an exercise metric of a fitness equipment unit.

## Description

### FIELD OF THE INVENTION

The present invention relates to an apparatus and method for generating codes associated with exercise metrics.

### BACKGROUND TO THE INVENTION

Many fitness equipment units have minimal data processing, data transmission and power capabilities. Such fitness equipment units have little or no capability for processing or outputting exercise metrics or exercise results. As a result it is difficult to incorporate the use of such fitness equipment units into an overall exercise program or exercise routine.

### SUMMARY OF THE INVENTION

An aspect of the present invention relates to an apparatus comprising:
a display; and
a controller to receive signals representing different values for an exercise metric of a fitness equipment unit and to geerate control signals causing the display to present different graphical codes based on the different values for the exercise metric.

The apparatus may comprise a sensor to sense an exercise metric of the fitness equipment unit. The sensor may comprise one or more a reed switches.

The display and the controller may be powered by a battery, The apparatus may comprise a photovoltaic cell to recharge the battery.

At least one, for example each, graphical code may comprise at least one quick response (QR) code.

An exercise metric represented by at least one graphical code may comprise motion of a movable member of the fitness equipment unit,

An exercise metric represented by at least one graphical code may comprise a number of repetitions of movement of a movable member of the fitness equipment unit.

An exercise metric represented by at least one graphical code may comprise a speed of movement of a moveable member of the fitness equipment unit.

An exercise metric represented by at least one graphical code may comprise a time duration of movement of a movable member of the fitness equipment unit.

An exercise metric represented by at least one graphical code may comprise a level of resistance to movement of a movable member of the fitness equipment unit.

An exercise metric represented by at least one graphical code may comprise which of a plurality of selectable weights of the fitness equipment unit has been selected.

At least one graphical code may indicate an identity of the fitness equipment unit.

At least one graphical code may identify a day on which exercise is performed.

At least one graphical code may represent at least two exercise metrics for the fitness equipment unit,

At least one graphical code may comprises a barcode.

The apparatus may comprise the fitness equipment unit.

The display and the controller may be removably to the fitness equipment unit.

The fitness equipment unit may comprise a stack of selectable weights to be repeatedly moved by a person during exercise. An exercise metric represented by at least one graphical code may comprise a selected weight of the stack of selectable weights. An exercise metric represented by at least one graphical code may comprise a number of times at which a selected weight has been lifted. An exercise metric represented by at least one graphical code may comprise a distance at which a selectable weight has been lifted.

The apparatus may comprise an input to indicate completion of an exercise. The controller may cause generation of at least one graphical code in response to the input indicating completion of the exercise.

The apparatus may comprise a code capture device to capture a digital image of at least one graphical code. The apparatus may comprise a non-transient computer-readable medium storing computer-readable code to translate the digital image of at least one graphical code into the different values for the exercise metric,

The apparatus may comprise a portable electronic device. The portable electronic device may include a code capture device and a non-transient computer-readable medium.

The apparatus may comprise a remote computing device.

The remote computing device may be configured to receive signals from a portable electronic device. The signals may represent one or more values for the exercise metric. The remote computing device may be configured to transmit messages for display by a portable electronic device, for example based upon one or more values for the exercise metric.

The apparatus may comprise a portable electronic device comprising a portable electronic device display, and a code reader, such as a barcode reader, QR code reader or the like, or any suitable combination.

An aspect of the present invention relates to a method, such as a method of using an apparatus as defined above.

The method may comprise receiving signals representing different values for an exercise metric of a fitness equipment unit. The method may comprise generating and displaying different graphical codes based on different values received for the exercise metric.

The method may comprise sensing an exercise metric of the fitness equipment unit.

The method may comprise supplying all power for the generating and displaying of the different graphical codes from one or more local batteries.

At least one graphical code may comprise a quick response (QR) code.

An exercise metric represented by at least one graphical code may comprise motion of a movable member of the fitness equipment unit.

An exercise metric represented by at least one graphical code may comprise a number of repetitions of movement of the movable member.

An exercise metric represented by at least one graphical code may comprises a speed of movement of a movable member of the fitness equipment unit.

An exercise metric represented by at least one graphical code may comprise a time duration of movement of a movable member of the fitness equipment unit.

An exercise metric represented by at least one graphical code may comprise a level of resistance to movement of a movable member of the fitness equipment unit.

An exercise metric represented by at least one graphical code may comprise which of a plurality of selectable weights of the fitness equipment unit has been selected.

At one graphical code may indicate an identity of the fitness equipment unit.

At least one graphical code may identify a day on which exercise is performed.

At least one graphical code may represent at least two exercise metrics for the fitness equipment unit.

At least one graphical code may comprises a barcode.

The fitness equipment unit may comprise a stack of selectable weights to be repeatedly moved by a person during exercise. An exercise metric represented by at least one graphical code may comprise a selected weight of the stack of selectable weights. An exercise metric represented by at least one graphical code may comprise a number of times at which a selected weight has been lifted. An exercise metric represented by at least one graphical code may comprise a distance at which a selectable weight has been lifted.

The method may comprise inputting an indication of completion of an exercise. The method may comprise the generation of at least one graphical code is in response to the input indicating completion of the exercise.

The method may comprise reading at least one graphical code with a code reader of a portable electronic device to translate the graphical code into the different values.

The method may comprise transmitting the different values to a remote computing device. The method may comprise receiving signals from the remote computing device that are based upon the different values. The method may comprise displaying a message on the portable electronic device based upon the received signals.

Features defined in relation to one aspect may be applied to any other aspect.

### BRIEF DESCRIPTION OF THE DRAWINGS

These and other aspects of the present invention will now be described, by way of example only, with reference to the accompanying drawings, in which:
Figure 1 is a schematic illustration of an example exercise metric output system;
Figure 2 is an example of a first graphical code that may be utilized with the exercise metric output system of Figure 1;
Figure 3 is an example of a second graphical code that may be utilized with the exercise metric output system of Figure 1;
Figure 4 is a flow diagram of an example method that may be carried out by the exercise metric output system of Figure 1;
Figure 5 is a schematic illustration of an example exercise system;
Figure 6 is a flow diagram of another example method that may be carried out by the exercise system of Figure 5;
Figure 7 is a flow diagram of another example method and corresponding content that may be carried out and displayed, respectively, by the exercise system of Figure 5;
Figure 8 is a diagram of an example graphical code and corresponding translated output of values for exercise metrics from a fitness equipment unit;
figure 9 is a schematic illustration of an example implementation of the exercise metric output system of Figure 1;
Figure 10 is a fragmentary schematic illustration of an example implementation of the exercise metric output system of Figure 8; and
Figure 11 is a schematic illustration of an example implementation of the exercise system of Figure 5.

### DETAILED DESCRIPTION OF THE EXAMPLE EMBODIMENTS

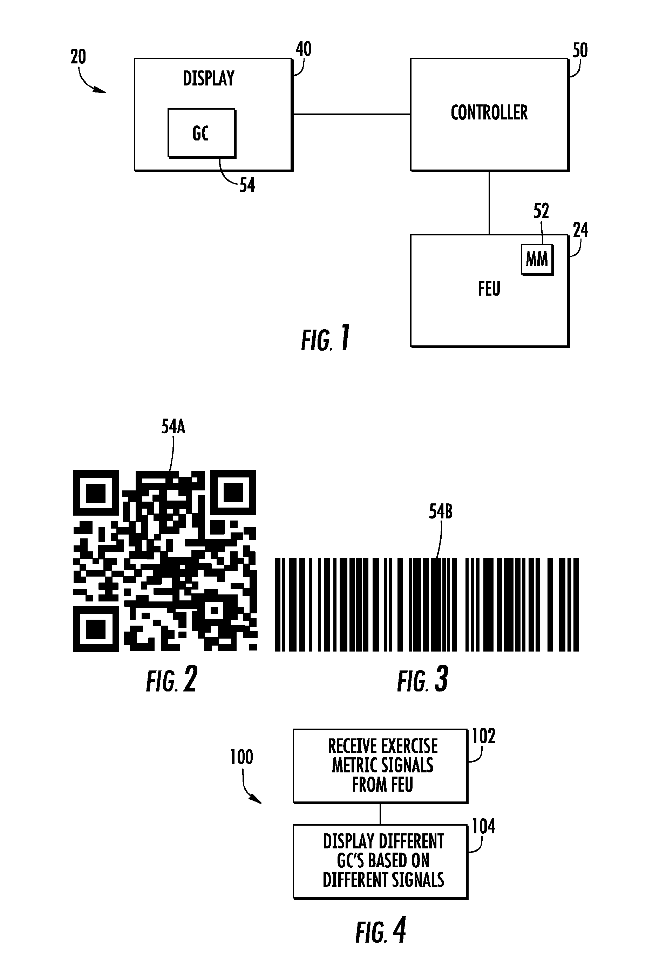

Figure 1 schematically illustrates an example exercise metric output system 20. As will be described hereafter, exercise metric output system 20 outputs values or results for exercise metrics for a fitness equipment unit with less power consumption or processing demands. Exercise metric output system 20 enables the output of values for exercise metrics from fitness equipment unit, a stand-alone fitness equipment unit, or a fitness equipment unit with no connection (LAN or Wi-Fi) to the Internet. Exercise metric output system 20 farther moves high-level software functions off of the fitness equipment unit 24 facilitating the use of low-power low-cost processors to drive a display. As a result, system 20 reduces the cost and complexity of electronics associated with the fitness equipment unit while also reducing power consumption to make alternative forms a power, such as solar or kinetic energy, feasible for supplying adequate amounts of power.

Exercise metric output system 20 comprises fitness equipment unit 24, display 40 and controller 50. Fitness equipment unit 24 comprises a machine or device with which a person interacts to carry out cardiovascular exercise, anaerobic exercise or combinations thereof. As schematically shown by Figure 1, fitness equipment unit 22 comprises one or more movable members 52, wherein each movable member 52 is adapted or configured to be contacted by an anatomy of a person to facilitate exercise by the person. In some implementations, an adjustable or controlled resistance may be applied against movement of movable member 52. In some implementations, the size, shape or inclination of a path through which the movable member 52 moves may be controlled or adjusted.

In one implementation, movable member 40 may comprise a footpad against which a person's foot or feet press against during exercise, Examples of fitness equipment units 24 or exercise devices that include such a footpad include, but are not limited to, elliptical machines, stepper machines, rowing machines, stationary bicycles, adaptive motion machines, ski simulation machines, and leg press machines. In one implementation, movable member 52 may comprise a belt against which the user contacts, such as those used in treadmills. In one implementation, movable member 52 may comprise a handgrip about which a person grasps to apply force during exercise. Examples of fitness equipment units 24 or exercise devices that include such handgrips include, but are not limited to, elliptical machines (swing arms), stepper machines (swing arms), adaptive motion machines (swing arms), climbing machines, pendulum motion machines, ski simulation machines, rowing machines, weight pull down machines, chest press machines and the like. In one implementation, movable member 52 may comprise a member configured to contact other portions of an anatomy such as members that contact a person's shins (leg press), a person's shoulders (squat machine), or a person thighs (abdominal exercise machine).

Display 40 comprises a display screen configured to present or display images of different graphical codes 54 that change from time-to-time depending upon different exercise metrics or exercise results. Depending upon the type of graphical code 54 to be displayed, display 40 may comprise a liquid crystal display, light emitting diode display, organic light emitting diode display (OLED), an electronic ink (e-ink) display or other types of display technology in present use or developed in the future.

Examples of a graphical code are shown in Figures 2 and 3. Figure 2 illustrates an example quick response (QR) code 54A. Figure 3 illustrates example one-dimensional barcode 54B. For purposes of this disclosure, a "graphical code" is a machine-readable graphic of lines, spots, bars, or symbols representing data a codified manner, wherein the graphic is not alphanumeric and wherein the data represented by the graphic is not readily identifiable or capable of being understood without being translated using a code key for the graphic. Other examples of graphical codes include, but are not limited to, a two-dimensional barcode, a three dimensional barcode and a circular guilloche. In other implementations, display 40 may be configured to dynamically present different images of other types of graphical codes currently in existence or developed in the future.

Controller 50 comprises one or more processing units configured to receive signals representing different values for an exercise metric of fitness equipment unit 24 and to control signals causing display 42 present different graphical codes based on different values for the exercise metric from the fitness equipment unit 24. For example, controller 50 generates control signals causing display 40 to present a first image of a first type of a graphical code, wherein the first image represents first results for the particular exercise metric. Controller 50 may generate control signals causing display 40 to present a second image, different than the first image, of the same first type of graphical code, wherein the second image represents second results, different than the first results, for the particular exercise metric. By way of a specific example, controller 50 may generate control signals causing display 40 to present a first graphical code, such as a first QR code 54A in response to receiving signals indicating that a person is lifting a first amount of weight, Controller 50 may generate control signals causing display 40 to present a second QR code 54A, different than the first QR code 54A, in response to receiving signals indicating that a person is lifting a second amount of weight different than the first amount of weight.

For purposes of this application, the term "processing unit" shall mean a presently developed or future developed processing unit that executes sequences of instructions contained in a memory. Execution of the sequences of instructions causes the processing unit to perform steps such as generating control signals. The instructions may be loaded in a random access memory (RAM) for execution by the processing unit from a read only memory (ROM), a mass storage device, or some other persistent storage. In other embodiments, hard wired circuitry may be used in place of or in combination with software instructions to implement the functions described. For example, controller 92 may be embodied as part of one or more application-specific integrated circuits (ASICs). Unless otherwise specifically noted, the controller is not limited to any specific combination of hardware circuitry and software, nor to any particular source for the instructions executed by the processing unit.

Figure 4 is a flow diagram of an example method 100 and may be carried out by exercise metric output system 20. As indicated by step 102, controller 50 receives signals representing different values for a least one exercise metric of a fitness equipment unit, such as fitness equipment unit 24. In one implementation, controller 50 receive signals representing different values for multiple exercise metrics of exercise on the fitness equipment unit 24. Examples of exercise metrics that may be represented by signals received by controller 50 include, but are not limited to, a speed at which movable member 52 is being moved, and acceleration of movable member 52, a time duration of movement of who member 52, a level of resistance against movement of movable member 52, a time of day or a date at which the exercise was initiated or completed, a number of repetitions or a number of sets of repetitions completed, and a range or distance through which movable member 52 has been moved.

Such signals may be generated using one or more sensors sensing movement of movable member 52 (or movement of other members or structures which move in response to movement of movable member 52). Such signals may also or alternatively be generated based upon selected value settings entered for exercise metrics on fitness equipment unit 24. For example, fitness equipment unit 24 may be configured to allow a person exercising to select one or more values for exercise parameters for exercise metrics on fitness equipment unit 24, wherein such values during an individual exercise session do not change or only change according to a predefined a predetermined program or control routine. In one implementation, fitness equipment unit 24 may comprise an exercise device, such as a treadmill, elliptical machine, stair stepper, and the like, wherein a person selects all the values for the particular exercise session on the exercise device, such as the peed, resistance, and duration, In such an implementation, the signals representing values for the exercise metrics may be generated directly from the selections of the values, wherein sensors are not needed to detect values for such exercise metrics. In such an implementation, the signals represent the selected values for the parameters are exercise metrics rather than sensed values.

As indicated by step 104, based upon such signals received in step 102, controller 50 generate control signals causing display 42 display different graphical codes 54. For example, during a first exercise session, a person may lift a first amount of weight, for a first number of repetitions, through a first distance, within a first period of time. Based upon such values, as represented by signals received from fitness equipment unit 24, controller 50 may cause display 40 to present a first image of a graphical code 54.

During a second exercise session, the person may lift a second amount of weight, for a second number of repetitions, through a second distance within a second period of time. Based upon such values for the second exercise session, as represented by signals received from fitness equipment unit 24, controller 50 may cause display 40 to prevent a second image, different than the first image, of the graphical code 54. For example, the first exercise session may result in a first QR code being presented on the second exercise session may result in a second QR code being presented.

Each of the first and second images of graphical code 54, for the first and second exercise sessions, may be captured by a camera, scanner or other code capturing device of a portable electronic device carried by the person exercising for subsequent translation, storage, analysis and use. Because system 20 and method 100 present a graphical code based upon different for one or more exercise metrics, such values are compactly presented for fast and efficient capture by a portable electronic device, allowing such graphical codes to be translated to the exercise metric values that they represent independent of system 20 or fitness equipment unit 24. The translated graphical codes may be analyzed, stored, used and transmitted independent of system 20 or fitness equipment unit 24.

Because the analysis, storage, use and transmission of the values for the one or more exercise metrics from fitness equipment unit 24 may be performed independent of fitness equipment unit 24 or system 20, system 20 and fitness equipment unit 24 may be simpler in construction and lower in cost. Rather than having to connect fitness equipment unit 24 to a substantial power source, such as an electrical outlet, controller 50 and display 40 have a relatively small, reduced power demand, allowing them to be operated using power from a battery or rechargeable battery. Rather than having to connect fitness equipment unit 24 in a wired or wireless fashion to a network, data transmission may be carried out using the portable electronic device that captured the graphical code. Rather than having to provide fitness community 24 with processing or memory capability to analyze and store the results of an exercise session, such analysis and storage of the results may alternatively be carried out remote from unit 24 on a portable electronic device or another computing device in communication with the portable electronic device.

System 20 further facilitates incorporation of existing fitness equipment unit 24 lacking substantial processing capabilities or power supplies into overall exercise routines or programs. Because the automatic programmed generation of graphical codes 54 based upon signals representing values of exercise metrics may be carried out with little processing and with a relatively small amount of power consumption, existing fitness equipment units lacking substantial processing, data transmission capability or power availability may be easily modified or adapted to be incorporated as part of a system 20. As a result, the results of an exercise session on such fitness equipment units may be evaluated under a fitness program and may be possibly utilized for adjustment of a fitness routine or program.

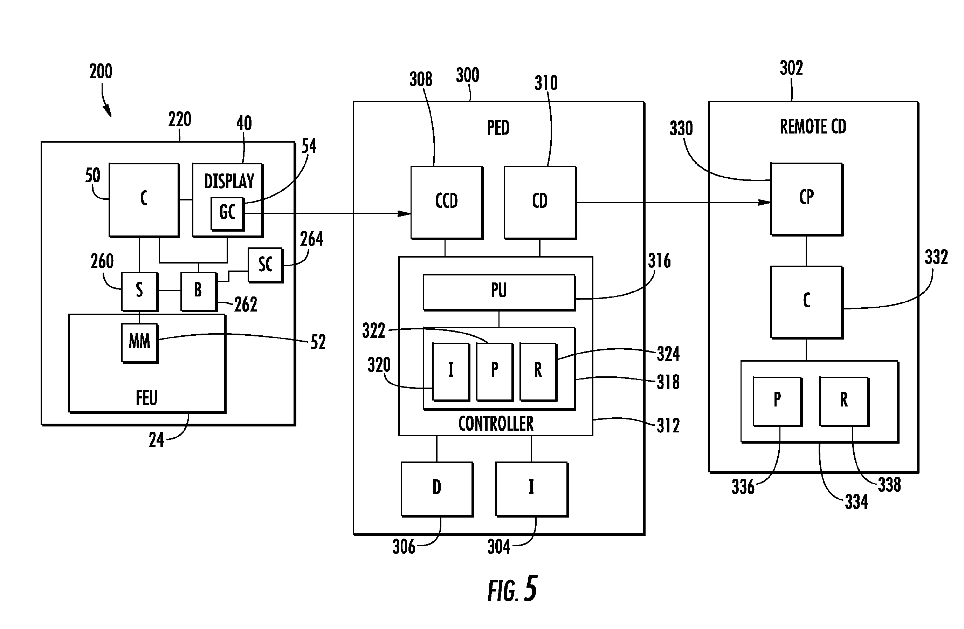

Figure 5 schematically illustrates exercise system 200. Exercise system 200 comprises exercise metric output system 220, an example implementation of exercise metric output system 20, portable electronic device 300 and remote computing device 302. Exercise metric output system 220 is similar to exercise metric output system 20 except that exercise metric output system 220 is specifically illustrated as including sensor 260, battery 262 and solar cell 264. Those remaining components of system 220 which correspond to system 20 are numbered similarly.

Sensor 26 comprises one or more sensors to detect movement of the one or more movable members 52. In one implementation, sensor 260 may comprise one or more sensors to sense or detect values for exercise parameters or metrics such as a speed at which movable member 52 is being moved, and acceleration of movable member 52, a time duration of movement of who member 52, a level of resistance against movement of move member 52, a time of day or a date at which the exercise was initiated or completed, a number of repetitions or a number of sets of repetitions completed, and a range or distance through which movable member 52 has been moved. In one implementation, sensor 260 may comprise a photo or optical sensor having a photo emitter and photodetector. In another implementation, sensor 260 may comprise a reed switch, wherein the switch is actuated in response to a changing magnetic field. In other implementations, sensor 260 may comprise other forms of sensors relying upon optics, magnetics and the like.

Battery 262 comprises a self-contained source of electrical power. Battery 262 supplies electrical power to sensor 260, display 40 and controller 50. In one implementation, battery 262 is movable replaceable. In one implementation, battery 262 is additionally rechargeable. Battery 262 facilitate use of up system 220 circumstances where connection to an electrical outlet is difficult or impossible. In some implementations, battery 262 may be omitted.

Solar cells 264 comprise one or more devices to capture or harness solar power for use in powering sensor 260, display 40 and controller 50 and/or for use in charging battery 262. In one implementation, solar cells 264 comprise photovoltaic cells. In other implementations, solar cells 264 may comprise other forms of solar or light harnessing devices for generating electrical power or charge for use by system 220. In other implementations, solar cells 264 may be omitted.

Portable electronic device 300 comprises a portable handheld electronic device configured to capture graphical code 54 presented on display 40. Examples of such a portable electronic device (PED) 300 include, but are not limited to, a smart phone, a personal data assistant (PDA), laptop, notebook computer, tablet computer (e.g. IPAD) and MP3 player (e.g., IPOD TOUCH). In the example illustrated, PED 300 comprises input 304, display 306, code capture device 308, communication device 310 and controlled 312. In other implementations, PED 300 may comprise a fewer or greater of such components so long as PED 300 includes code capture device 308.

Input 304 comprises a user interface for PED 300 by which inputs are made to PED 300. Input 304 facilitates entry of inputs or commands by a person to initiate the capture of graphical code 54 by code capture device 308 and two initiate transmission of the captured graphical 54 or in a translated graphical code 54 by communication device 310. Input 304 may additionally or alternatively facilitate viewing of a translated graphical 54 or the viewing of an overall exercise routine or program, exercise instructions or modifications to the overall exercise routine or program based upon the translated graphical code 54 representing values or results for an exercise metric of an exercise session that has just been completed. In one implementation, input 304 comprise a keyboard. In other implementations, input 304 may comprise a touchpad, a stylus, a microphone with associated speech recognition software or programming, a touch screen, buttons, switches and the like. In some implementations, input 304 may comprise a touch screen incorporated as part of display 306.

Display 306 comprises a user interface by which graphical or textual data is presented to a user. In one implementation, display 306 comprises a liquid crystal display. In other implementations, display 306 may comprise other types of display technology such as a light emitting diode display, an organic light emitting diode display (OLED), an electronic ink (e-ink) display or other types of display technology it present use or developed in the future.

Code capture device 308 comprises a device configured to capture or read the image of the graphical code 54 presented on display 40 (without being physically connected to controller 50). In one implementation, code capture device 304 comprises a camera, such as a camera including charge coupled devices or sensors arranged in a two-dimensional array or a cell phone or smart phone camera. In other implementations, code capture device 308 may comprise a charge coupled device reader, a pen-type reader comprising a light source and photo diode detecting the intensity of light reflected from graphical code 54, a laser scanner using a photo diode to measure intensity of light reflected from graphical code 54, an omnidirectional scanner or other types of presently use or future developed image capturing devices.

Communication device 310 comprises device configured to facilitate the transmission of the captured graphical code 54 or a translation of the captured graphical code 54 to external destinations such as remote computing device 302. In some implementations, communication device 310 additionally facilitates the receipt of translated graphical code 54, after graphical 54 has been transmitted to an external or remote recipient or server that performs the translation. In one implementation, communication device 3 110 facilitates the receipt of instructions or overall workout routine adjustments that are based in part upon the values represented by the graphical code 54.

In one implementation, communication device 310 comprises a wired port or wired connection. In another implementation, communication device 3 and 10 comprises a wireless communication device configured to transmit data in a wireless fashion. In some implementations where communication device 310 itself translates and utilizes graphical code 54, communication device 310 may be omitted.

Controller 312 comprises generate control signals directing the operation of portable electronic device 300. Controller 312 comprises processing unit 316 and memory 318. Processing unit 316 receives graphical code 54. In one implementation, processing unit 316 facilitates forwarding of the received graphical code 54 by communication device 310 for translation in use remote from PED 300. In another implementation, processing unit 316 translates graphical code 54 to the one or more values for the one or more exercise metrics prior to forwarding the translated graphical code using communication device 310. In yet other implementations, processing unit 316 translates graphical code 54 and utilizes the translated graphical 54 in one or more fashions such as storing the translated graphical code 54, providing encouragement or suggesting workout changes communicated through display 306 based upon the translated graphical code 54 and the values for the exercise metrics. In some implementations, processing unit 316 may simply store the untranslated graphical code 54, wherein the untranslated graphical code 54 is later retrieved when PED 300 is subsequently placed in communication with an external recipient that is to translate the graphical code 54.

Memory 318 comprises a non-transient computer-readable medium or persistent storage device configured to store software, programming, computer readable instructions and/or data. In the example illustrated, memory 318 stores instructions 320, exercise program 322 and results 324. Instructions 320 comprise computer-readable code or programming configured direct the operation of processing unit 316. In one implementation, such instructions direct processing unit 316 in the translation of graphical code 54 to the values for the exercise metrics being represented by graphical code 54. Such instructions 320 further direct the other various operations for processing unit 316 described above as well as other operations for processing unit 316 pertain to the general use of PED 300.

In one implementation, part of such instructions 320 may comprise a downloaded application for collecting graphical codes and manipulating the information from the graphical codes. For example, the supplier or manufacturer of system 220 may offer an application that exercisers may download for this purpose. Although some smartphones IPODs or other PEDS may have QR Code readers or other graphical code readers, they may still lack specialized software or an application that is targeted toward manipulating the data/information from the QR codes or other graphical codes that are read by the smart phone, IPOD or other PED.

Exercise programs 322 comprise one or more applications or programs indicating recommended settings or workout parameters to be used by a person exercising so as to achieve an exercise objective or exercise or fitness target or goal. Using the data contained in such exercise programs 322, controller 316, following instructions 320, displays various recommendations using display 306. In some implementations, the values or parameters for an exercise routine or program may be adjusted based upon translated graphical codes 54 (actual exercise results or exercise values indicated by the translated graphical codes 54). In some implementations, such exercise programs may not be stored on PED 300.

Results 324 comprise stored results from an exercise session. In one implementation, results 324 may comprise stored translated graphical codes 54 (the actual exercise values for the exercise metrics). In another implementation, results 324 merely comprise the untranslated graphical code 54, wherein the untranslated graphical code 54 is to be subsequently translated. In some implementations, results 324 may not be stored in memory 318 of PED 300.

Remote computing device 302 comprises one or more computing devices remote with respect to fitness equipment unit 24. In one implementation, remote computing device 302 may comprise a computing device at a fitness facility separate from fitness equipment unit 24. In another implementation, remote computing device 302 may comprise a computing device, such as a server, remote from the fitness facility housing or containing fitness equipment unit 24. In one implementation, remote computing device 302 may be provided at a central facility servicing multiple fitness facilities at multiple geographic locations. In one implementation, the central facility may be provided by a center for an organization of multiple fitness facilities. In another implementation, the central facility may be provided by the manufacturer or supplier of fitness equipment unit 24, providing enhanced capabilities for both fitness communities 24 provided by the manufacturer or supplier of the fitness equipment unit 24. In still other implementations, remote computing device 302 may be provided as part of another fitness equipment unit distinct from fitness community 24, wherein the other fitness equipment unit has the higher end computing , data storage or data transmission capability for processing, storing and transmitting the translated or untranslated graphical code 54. For example, in one implementation, fitness or query unit 24 may be an unpowered standalone weightlifting device, wherein PED 300 may transfer the displayed results in the form of graphical code 54 to another exercise machine, such as an elliptical machine treadmill, stair stepper, adaptive motion machine and the like, which has the ability to store, process or transmit the results from the weightlifting device represented by the graphical code 54.

In the example illustrated, remote computing device 302 comprises communication device 330, controller 332 and memory room 334 storing an exercise programs 336 and exercise results 338. Communication device 330 comprises a device configured to facilitate communication between remote computing device 302 and portable electronic device 300. The configuration communication device 330 may depend upon the configuration of communication device 310. Communication device 330 facilitates the receipt of either an untranslated graphical code 54 from PED 300 or a translated graphical code 54 from PED 300, depending upon the implementation. Communication device 330 further facilitates the transmission of a translation of a received untranslated graphical code 54 to FED 300 and/or the transmission of exercise routine adjustments to PED 300 based upon the values represented by graphical code 54. In one implementation, communication device 330 comprises a wired port or wired connection. In another implementation, communication device 330 comprises a wireless communication device configured to transmit data in a wireless fashion.

Controller 332 comprises one or more processing units configured to carry out operations of server 302. In one implementation, controller 332, following instructions contained in memory 334, may be configured to receive an untranslated graphical code 54 and carry out a translation of the graphical code 54 to the corresponding values for the one or more exercise metrics. In one implementation, controller 332 basically receive already translated graphical codes 54. Using the values represented by the graphical codes 54, controller 332 may adjust existing or ongoing exercise programs or routines stored in memory 336. Controller 332 may farther store the exercise values (results 338). As a result, controller 3 through two may provide access to such results to other portable electronic devices or through other computing devices connected to server 302 across a network, such as a local area network or the Internet.

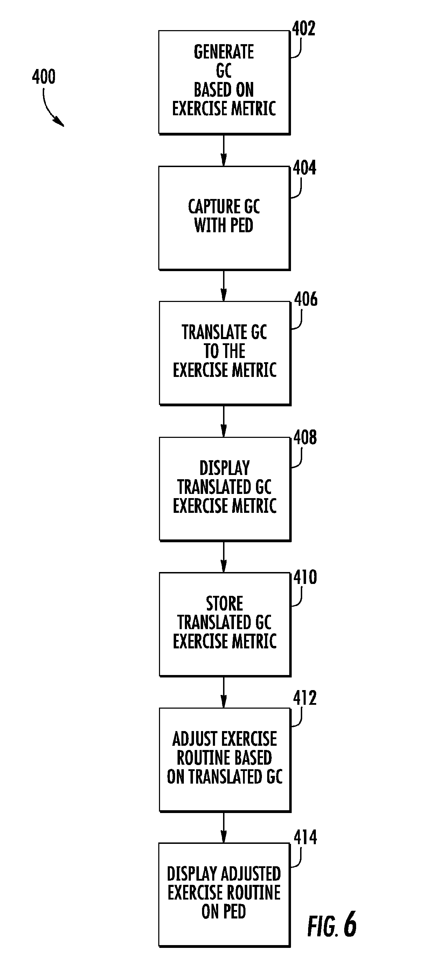

Figure 6 is a flow diagram of an example method 400 that may be carried out by system 200. As indicated by step 402, controller 50 generates graphical code 54 based upon values for one or more exercise metrics received either from sensor 260 or from fitness equipment unit 24 (such as where the values are user selected parameter settings which are not sensed). In one implementation, in addition to being based upon the values for the one or more exercise metrics, graphical code 54 may additionally be based upon other information such as the identity of the fitness equipment unit 24 and/or the identity or characteristics pertaining to the person exercising.

As indicated by step 404, in response to input through input 304, processing unit 316 directs code capture device 308 to capture the image of the generated graphical code 54. Such may be achieved by the person positioning the lens or other opening of the code capture device 308 in close proximity to and opposite to display 40 while display 40 is presenting the image of the graphical code 54. User may then initiate the captured using input 304, similar to a person opening the shutter of a camera.

As indicated by step 406, the captured graphical code 54 is translated to the one or more values for the one or more exercise metrics. In one implementation, such translation may be carried out by controller 312. In another implementation, such translation to be carried out by a remote computing device 302 after the untranslated graphical code 54 has been transmitted thereto. In such a case, the remote computing device 302 transmits the translated graphical code 54 (the values for the one or more exercise metrics) back to PED 300.

As indicated by step 408, the translated graphical code (the values for the one or more exercise metrics of the particular exercise session) is displayed for review by the person who had adjust completed the exercise session. In one implementation, the values are displayed on display 306 by processing unit 316. As a result, person exercising may see a tally of the complete exercise session that has been captured. For example, the person may view the number of repetitions, the time to complete the number of repetitions, the resistance or weight of each repetition and/or a distant through which each weight has been moved through one or more of the repetitions. In other implementations, values for other exercise metrics may be displayed. In some implementations, step 408 may be omitted.

As indicated by step 410, the translated graphical code 54 (the values for the one or more exercise metrics of the particular exercise session) is stored. In one implementation, the values are stored as results 324 in memory 312 by processing unit 316. In another implementation, the values may alternatively or additionally be stored us results 338 in memory 334 by controller 332.

As indicated by step 412, the translated graphical code or codes 54 are used to adjust the overall exercise routine or fitness program. In one implementation, the translated graphical code or codes 54 are used by processing unit 316, following instructions 320, to adjust one or more exercise routines or programs 322 stored in memory 312. In another implementation, the translated graphical code or codes 54 are used by controller 332 at remote computing device 302 to adjust one or more exercise routines or programs 336 stored in memory 334. In yet other implementations, step 412 may be omitted.

As indicated by step 414, PED 300 displays the adjusted exercise routine or program on display 306. As a result, the person exercising is advised as how to adjust remaining exercise sessions on the same fitness equipment unit 24 or how to adjust exercise parameters or exertion levels on other fitness equipment units which are part of the overall fitness program. In some implementations, step 414 may be omitted.

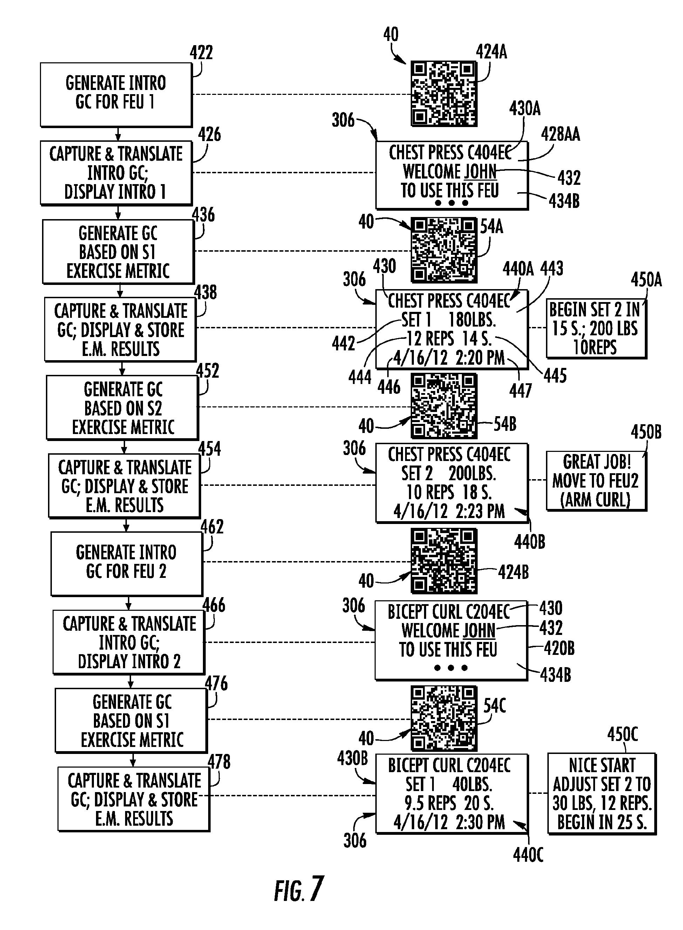

Figure 7 is a flow diagram of an example method 420 that may be carried out by system 200. The left side of Figure 7 illustrates various steps of method 420 while the right side of Figure 7 illustrates examples of corresponding resulting visible displays of such steps. Figure 7 illustrates an example wherein results for various exercise metrics associated with weightlifting machines or weightlifting fitness equipment units is output storage and use. In other implementations, the steps identified on the right side of Figure 7 may be carried out with other fitness equipment units. In other implementations, less than all of the illustrated steps on the right side of Figure 7 may be carried out.

As indicated by step 422, controller 50 generates an introductory graphical code and generates control signals causing display 40 to visibly present the graphical code (exemplified on the right side of Figure 7 with graphical code 424 shown as a QR code). In one implementation, the generation and display of the introductory graphical code 424A may be continuous while the particular fitness equipment unit 24 is not being used. In another implementation, the generation and display of the introductory graphical code 424 may occur in response to the sensed presence of a person in proximity to fitness equipment unit 24. In yet another implementation, the generation and display of the introductory graphical code 424A may occur in response to a person entering his or her identification to a reader or other input associated with fitness equipment unit 24 (when so equipped). In such an implementation, the person's identification may be codified into the presented graphical code 424A, allowing the person to confirm the proper entry of his or her identification information.

As indicated by step 426, code capture device 308 captures an image of the intro graphical code 424A displayed on display 40. As further indicated by step 426, the captured intro code 424A is then translated and displayed on display 306. In one implementation, processing unit 316, following instructions 320, translates the intro graphical code 424A, the translation of which is then displayed on display 306. In another implementation, the captured intro graphical code may be transmitted to another computing device, such as remote computing device 302 which performs the translation and returns the translated information back to FED 300 for display by display 306.

As shown on the corresponding right side of Figure 7, the translation of graphical code 424A results in the presentation of an introductory or instructional message on display 306. In the example illustrated, the message 428A produced in response to a translation of graphical code 424A comprises a fitness equipment unit identifier 430A, a user identification confin-nation 432 and an instructional portion 434A. The fitness equipment unit identifier 430A identifies the type of fitness equipment unit ("chest press" in the example) as well as a specific SKU identifier (number C404EC) specifically identifying the particular chest press machine. Other specific identifier such as a serial number, and also be used as part of the specific identifier for the fitness equipment unit identified in message 428A. The user identification confirmation 432 has a greeting portion along with an identification of the user about to use the particular fitness equipment unit as input by the person at the particular fitness equipment unit or as by otherwise determined by the fitness equipment unit. As noted above, in some implementations, they identification of the person about to use a particular fitness equipment unit may be omitted, The instructional portion 434A provides instructions as to how to use the particular fitness equipment unit, providing information such as proper technique or safety precautions.

In other implementations, the information presented as a result of the translation of the intro graphical code 424A may comprise less information or greater amounts of the same or other information. In one implementation, the contents of message 428A are entirely derived from the translation of the introductory graphical code 424A. In other words, all of the information of message 428A is codified as part of graphical code 424A. In another implementation, the translation of introductory graphical code 424A merely comprises an address (either a local address in the memory 318 of PED 300 or a remote address such as an address in memory 334 a remote computing device 302), wherein PED 300 utilizes the address from the translation to retrieve and display the contents of message 428A. In yet another implementation, the contents of message 428A may be derived from both the translation of graphical code 424A and from information retrieved from an address identified by the translation of graphical code 424A. In some implementations, steps 422 and 426 may be omitted.

Steps 436 and 438 substantially correspond to steps 402-410 of method 400. As indicated by step 436, controller 50 receives signals representing different values for one or more exercise metrics and generates graphical code 54 based upon such values for the one or more exercise metrics. As indicated by step 438, code capture device 308 captures an image of the dynamic exercise metric graphical code 54A (an example of graphical code 54 described above) displayed on display 40. As further indicated by step 426, the captured code 54A is then translated and displayed on display 306. In one implementation, processing unit 316, following instructions 320, translates the graphical code 54A, the translation of which is then displayed on display 306. In another implementation, the captured intro graphical code may be transmitted to another computing device, such as remote computing device 302 which performs the translation and returns the translated information back to PED 300 for display by display 306.

As shown on the corresponding right side of Figure 7, the translation of graphical code 54A results in the presentation of exercise results 440A on display 306. In the example illustrated, the presented exercise results 440 comprises a fitness equipment unit identifier 430 (described above), an exercise grouping or set identifier 442, the resistance or weight metric value 443, a distance or repetition metric value 444, and elapsed time or duration metric value 445, a date stamp value 446 and a timestamp value 447. The exercise grouping or set identifier 442 identifies particular grouping or set/subset of the exercise session for which results are being presented.

Metric value 443 comprise a resistance value for the exercise. In the example illustrated, the value is 180 pounds, which may read by the amount of resistance or the amount of weight being moved each repetition. In other fitness equipment units, the resistance may be the amount of is applied against movement of a movable member, such as against movement of a pedal or other structure. Such a metric value may be preset as by the machine or user or may vary. In implementations where the value of the resistance may vary during a single unit, such as during a single repetition, the value 443 may represent a statistical value, such as a mean or average of the resistance during individual unit or repetition.

Metric value 444 indicates the number of units completed during a particular set or exercise session, In the example illustrated in which the exercise is lifting weights on a chest press, value 444 identifies a number of units (repetitions) in the particular set 442. With other types of fitness equipment units, this metric may alternatively comprises a distance, such as the number of miles run, road, climbed or otherwise traversed.

Duration metric value 445 comprises an elapsed amount of time consumed during completion of the number of units are repetitions. In the example illustrated, metric value 445 (14 seconds) identifies the elapsed time you lies completing the 12 repetitions, In other implementations with other fitness equipment unit, duration metric 445 may indicate the elapsed time consumed during completion of the units 444, such as the amount of time consumed to complete a run of a certain number of miles.

Date stamp 446 and timestamp 447 identify the date and time at which an exercise session is taking place. In one implementation, such values may correspond to the time at which an exercise session is started. In another implementation, such values may correspond to the time at which an exercise session is completed. In other implementations, one or more of the pieces of information illustrated in the example result 440A may be omitted. In some implementations, other information may also be presented as part of results 440A.

As further shown on the corresponding far right side of Figure 7, in sone implementations, processing unit 316, following instructions 320 and program 322, may additionally present exercise routine or exercise program instructions 450A on display 306. Such instructions 450A may be present concurrently or sequentially with respect to results 440A. In the example, such instructions 450A are adjusted are based upon results 443. Such instructions 450A may provide encouragement as well as instruct the person exercising as to which one or more user selectable parameters or metrics values should be used during the next exercise session or sub session (set) and when the next session or set should be initiated. In the example illustrated, the obstruction 450A instructs the user to begin the next set in 15 seconds with a user selected resistance of 200 pounds and with a target or goal of 10 units or repetitions in the set. Depending upon exercise routine, such instructions may vary.

Steps 452 and 454 are carried out when the person exercising completes a second group or set on the same fitness equipment unit 24. Step 4 and 52 4 and 54 are identical to steps 436 and 438 except that they are carried out respect to the second group or set of exercise units (repetitions) carried out on the same fitness equipment unit. As illustrated on the right side of Figure 7 corresponding to step 452, controller 50 (shown Figure 5) generated graphical code 424B (a QR code in the example illustrated) based upon signals representing values one or more exercise metrics during the second set.

As illustrated on the right side of Figure 7 corresponding to step 454, the capture and translation of the graphical code 4248 results in the example results 440B being displayed on display 306 and being stored in one or more memories. In the example illustrated, results 440B include values for the same metrics as presented in results 440A. In the example illustrated, results 440B indicate that during set 2 on chest press C404EC, the user listed 200 pounds for 10 reps during a time period 18 seconds. Set 2 took place on April 16, 2012 and occurred (initiated or completed, depending upon the particular implementation) at 2:23 PM. As further illustrate on the right side Figure 7, processing unit 316 may additionally present instructions 450B. Instruction 450B and be based upon an evaluation or analysis of the just completed results 440B with respect to the overall exercise program or whether exercise targets or objectives for the particular session, day, week or month have or are being met. The present case, the objectives were met, resulting in praise. In addition, instruction 450B may instruct the person as to what exercise machine should be utilized next pursuant to program 322 on PED 300 (shown in Figure 5). In other implementations, instructions 450B may be omitted.

Steps 462, 466, 476 and 478 essentially correspond to steps 422, 426, 436 and 438, respectively, but for being carried out with respect to a different fitness equipment unit of fitness equipment machine. In the example illustrated, such steps are carried out with respect to a fitness equipment unit (FEU2) comprising an arm curl machine. As illustrated on the right side of Figure 7 corresponding to step 462, controller 50 of the arm curl fitness equipment unit 24 generates control signals causing introductory graphical code 4248 to be displayed on display 306 of the arm curl fitness equipment unit 24. Introductory graphical code 424 and its presentation are identical to the presentation of graphical code 424A above except that graphical code 424B comprises codified information pertaining to the second fitness equipment unit, the arm curl fitness equipment unit.

As indicated by step 466, code capture device 308 captures an image of the intro graphical code 424B displayed on display 40. As further indicated by step 466, the captured intro code 424B is then translated and displayed on display 306. In one implementation, processing unit 316, following instructions 320, translates the intro graphical code 424B, the translation of which is then displayed on display 306. In another implementation, the captured intro graphical code may be transmitted to another computing device, such as remote computing device 302 which performs the translation and returns the translated information back to PED 300 for display by display 306.

As illustrated on the right side of Figure 7 corresponding to step 466, the translation of graphical code 424 results in the presentation of an introductory or instructional message on display 306. In the example illustrated, the message 428B produced in response to a translation of graphical code 424B comprises a fitness equipment unit identifier 430B, a user identification confirmation 432 and an instructional portion 434B. The fitness equipment unit identifier 430B identifies the type of fitness equipment unit ("bicep curl" in the example) as well as a specific identifier (number C204EC) specifically identifying the particular bicep curl machine. Other specific identifier such as a serial number, and also be used as part of the specific identifier for the fitness equipment unit identified in message 428B. The user identification confirmation 432 has a greeting portion along with an identification of the user about to use the particular fitness equipment unit as input by the person at the particular fitness equipment unit or as by otherwise determined by the fitness equipment unit. As noted above, in some implementations, the identification of the person about to use a particular fitness equipment unit may be omitted, The instructional portion 434B provides instructions as to how to use the particular fitness equipment unit, providing information such as proper technique or safety precautions.

In other implementations, the information presented as a result of the translation of the intro graphical code 424B may comprise less information or greater amounts of the same or other information. In one implementation, the contents of message 428B are entirely derived from the translation of the introductory graphical code 424B. In other words, all of the information of message 428B is codified as part of graphical code 424B. In another implementation, the translation of introductory graphical code 424B merely comprises an address (either a local address in the memory 318 of PED 300 or a remote address such as an address in memory 334 a remote computing device 302), wherein PED 300 utilizes the address from the translation to retrieve and display the contents of message 428B. In yet another implementation, the contents of message 428B may be derived from both the translation of graphical code 424B and from information retrieved from an address identified by the translation of graphical code 424B. In some implementations, steps 462 and 466 may be omitted.

Steps 476 and 478 identical to steps 436 and 438, respectively, except that steps 476 and 478 pertain to the outputting and capturing of values or results for one or more exercise metrics on the second fitness equipment unit, the arm curl machine in the example. In the example, As indicated by step 476, controller 50 receive signals representing different values for one or more exercise metrics and generates graphical code 54C based upon such values for the one or more exercise metrics. As indicated by step 478, code capture device 308 captures an image of the dynamic exercise metric graphical code 54C (an example of graphical code 54 described above) displayed on display 40. As further indicated by step 476, the captured code 54C is then translated and displayed on display 306. In one implementation, processing unit 316, following instructions 320, translates the graphical code 54C, the translation of which is then displayed on display 306. In another implementation, the captured intro graphical code may be transmitted to another computing device, such as remote computing device 302 which performs the translation and returns the translated information back to PED 300 for display by display 306.

As shown on the corresponding right side of Figure 7, the translation of graphical code 54C results in the presentation of exercise results 440C on display 306. In the example illustrated, the presented exercise results 440C comprises a fitness equipment unit identifier 4308 (described above), an exercise grouping or set identifier 442, the resistance or weight metric value 443, a distance or repetition metric value 444, and elapsed time or duration metric value 445, a date stamp value 446 and a timestamp value 447. In the example illustrated, results 440C indicate that during set 1 on arm or bicep curl C204EC, the user listed 40 pounds for 9.5 reps during a time period 20 seconds. Set 1 took place on April 16, 2012 and occurred (initiated or completed, depending upon the particular implementation) at 2:30 PM.

In the example illustrated, a partial repetition may mean that the repetition was not complete. In other words, during one of the repetitions, the resistance or weight being lifted (40 pounds) was not moved through a distance (or angle) sufficient to qualify as a complete repetition. For example, the weights being lifted may only been lifted half the distance (or angle) sufficient (greater than a predefined threshold distance) to constitute a full arm curl repetition. In essence, results 224 indicate a value for a distance metric through which the weight has been moved. In other implementations, the results, as translated from a graphical code 54, may specifically identify a numerical value for such distance rather than a percentage of a predefined threshold distance for a unit, For example, a distance for arm curl may be identified by the degrees of an arc through which the weight is been curled. A distance for a press may be the vertical linear distance through which a weight has been moved.

As further shown on the corresponding far right side of Figure 7, in some implementations, processing unit 316, following instructions 320 and program 322, may additionally present exercise routine or exercise program instructions 450C on display 306. Such instructions 450A may be present concurrently or sequentially with respect to results 440A. In the example, such instructions 450A are adjusted are based upon results 440C. Such instructions 450C may provide encouragement as well as instruct the person exercising as to which one or more user selectable parameters or metrics values should be used during the next exercise session or sub session (set) and when the next session or set should be initiated. In the example illustrated, the obstruction 450C instructs the user to begin the next set in 25 seconds with a user selected resistance of 30 pounds and wish a target or goal of 12 units or repetitions in the set, Depending upon exercise routine, such instructions may vary.

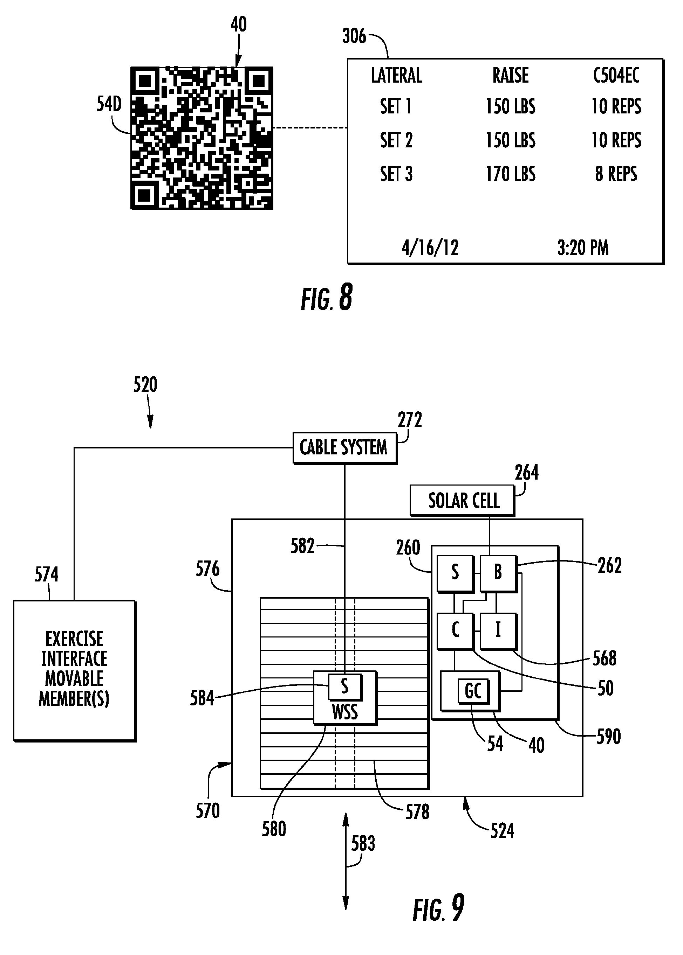

In other implementations, a single graphical code 54 may provide information for multiple sets on a particular exercise machine or fitness equipment unit 24. Figure 8 illustrate an example graphical code 54D along with the translated content of graphical code 54D as presented on display 306. In the example illustrated, the translation of graphical code 54D results in information being presented and stored which indicates that the exercise was performed on a lateral raise fitness equipment unit with an SKU of C504E. The information or content further indicates that a first set of 10 repetitions at 150 pounds was completed, a second set of 10 repetitions at 150 pounds was completed and a third set of 8 repetitions at 170 pounds was completed. The translated content indicates that the exercise took place on April 16, 2012 at 3:20 PM. In such an implementation, steps 436, 438, 452 and 454 may be consolidated into two steps: a step for generating a single graphical code such as graphical code 54D and a step for capturing and translating the graphical code whilst displaying of storing the results for the multiple sets of repetitions.

Figure 9 schematically illustrates exercise metric output system 520, an example implementation of exercise metrics output systems 20 and 220. Exercise metric output system 520 is similar to output system 220 except that output system 520 is specifically illustrated as comprising fitness equipment unit 524 for fitness equipment unit 24 and as additionally including input 568. Those remaining components or elements of a system 520 which correspond to elements of output system 220 are numbered similarly.

Fitness equipment unit 524 comprises weight system 570, cable system 572 and exercise interface movable members 574. Weight system 570 comprises a system providing a plurality of selectable weight that may be utilize and ultimately lifted in an exercise. Weight system 570 generally includes frame 576, a stack of individually selectable weights 578, weight selection System 580 and weight lift 582.

Frame 576 comprises one or more structures movably supporting and guiding the stack of weights 578. Weights 578 comprise structures having predetermined weight amounts which are configured to be raised and lowered in the direction indicated by arrows 583 so as to provide a mechanical resistance in an exercise. In the particular example illustrated, weights 578 each comprise a solid or hollow plate of one or more metals. In other embodiments, weight 30 may comprise other materials or may comprise encapsulated materials, such as sand, water or other materials. Weights were 578 are stacked upon one another such that as a particular weight 578 is being lifted, other weights 578 stacked upon the particular weight 578 are also lifted.

Weight selection system 580 comprises a mechanism configured to permit a person to select one or more of weights 578 for lifting during an exercise. Weight selection system 580 includes a selector 584 configured selectively couple one of the weights 578 to weight lift 582. In one implementation, selector 584 may comprise a pin movably positioned within a corresponding cavity of a selected one of weights 578 and into a corresponding aperture in weight lift 582. In other implementations, selector 584 may have other configurations.

For purposes of this disclosure, the term "coupled" shall mean the joining of two members directly or indirectly to one another. Such joining may be stationary in nature or movable in nature, Such joining may be achieved with the two members or the two members and any additional intermediate members being integrally formed as a a single unitary body with one another or with the two members or the two members and any additional intermediate member being attached to one another. Such joining may be permanent in nature or alternatively may be removable or releasable in nature. The term "operably coupled" shall mean that two members are directly or indirectly joined such that motion may be transmitted from one member to the other member directly or via intermediate members.

Weight lift 582 comprises a structure coupled to weight selection system 580 which is connected to cable system 572. In one embodiment, weight lift 582 may itself comprise a shaft or rod connected to a cable, in other implementations, weight lift 582 may have other configurations.

Cable system 572 comprises a system of pulleys and cables configured to operably couple weight lift 582 (and any connected weights 578) to exercise interface movable members 574. Cable system 572 may have any of a variety of different sizes, shapes and configurations depending upon exercise interface movable members 574. In other embodiments, exercise interface movable members 574 may be operably coupled to weight system 570 by other mechanisms.

Exercise interface movable members 574 comprise devices or mechanisms operably coupled to cable system 572 by which one or more persons may exert force against one or more structures and may move the one or more structures to raise or lift a selected amount of weight provided by weights 30 578. Exercise interface movable members 574 may have various configurations depending upon which particular muscles or groups of muscles are to be exercised. Examples of exercise interface movable members 574 include, but are not limited to the following types of exercise machines: abdominal isolator, angled seated calf, abductor, seated leg curl, glute isolator, vertical and horizontal, rear delt/pec fly, lateral raise, shoulder press, vertical press, back extension, seated row, vertical row, pull down, long pull, seated dip, seated tricep extension, bicep curl, camber curl and chest press. Exercise interface movable members 574 may be provided as part of a multi-station exercise machine, a modular exercise machine or a single station exercise machine.

As shown by Figure 9, solar cell 264, which recharges battery 262, is mounted to a top or upwardly facing surface of frame 576. As a result, solar cell 264 is best suited to capture light, from overhead lighting, for supplying the relatively low power demands of system 520. As further shown by Figure 9, in the example illustrated, display 40, controller 50, sensor 260, battery 262, solar cell 264 and input 568 are provided as a self-contained module 590 adapted to be mounted to frame 576. As a result, module 590 enable a pre-existing stand-alone fitness equipment unit 524 to be easily modified for outputting values for one or more exercise metrics.

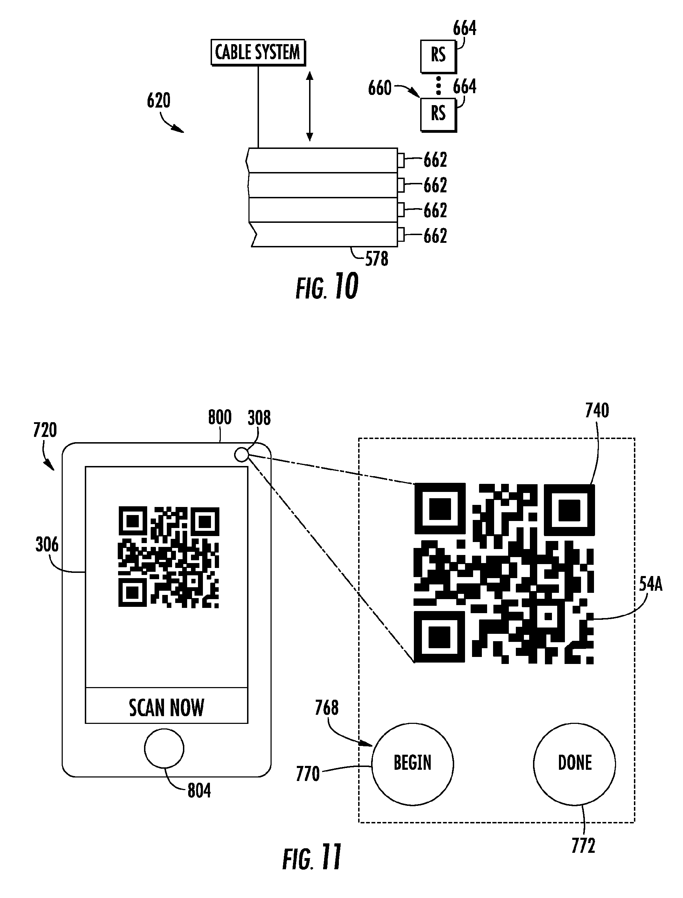

Figure 10 schematically illustrates exercise metric output system 620, the particular example of System 520. Exercise metric output system 620 is identical to exercise metric output system 520 except that system 620 specifically includes sensor 660. Sensor 660 comprises magnets 662 and reed switches 664. Magnets 662 comprise individual magnetic members, each magnet 662 carried by an associated weight 578. Magnets 662 have a sufficiently strong magnetic field so as to actuate reed switch 664 as such magnets 662 are moved past and across reed switch 664.

Reed switches 664 comprise electrical switches supported by frame 576 proximate to and vertically along weights 578 and their associated magnets 662. Each reed switch 664 is operated by an applied magnetic field, wherein the magnetic field (from an electromagnet or a permanent magnet) causes reeds of switch 664 to come together, thus completing an electrical circuit. The stiffness of the reeds causes them to separate, and open the circuit, when the magnetic field ceases. In another implementation, a non-ferrous normally-closed contact that opens when the ferrous normally-open contact closes may be utilized. In yet other implementations, reed switch 664 may utilize mercury "wetted" contacts. In operation, weights 662 are raise or lowered past a reed switch 664, an electrical circuit is repeatedly opening closed to indicate the number of weights 578 being lifted. In the example illustrated, because sensor 660 includes multiple vertically arranged reed switches 664, the distance at which weights 578 is lifted may also be detected. In other implementations, sensor 660 may comprise a single reed switch 664.

Figure 11 illustrates exercise metric output system 720, another example implementation of exercise metric output system 220. System 720 is identical to system 220 except that system 720 specifically includes display 740, input 768 and portable electronic device 800. In the example illustrated, input 768 comprises a start or begin button 770 and a finished, complete or done the button 772. When a person is to begin ain exercise session for which a graphical code is to be generated, the person to exercise presses or otherwise actuates the begin button 770. This results in the sensing and transmission of signals representing values of one or more exercise metrics to begin. When the person exercising presses the done button 772, such signal transmission is terminated or such signals are no longer used in the generation of a graphical code, In one implementation where weights are repeatedly lifted (repetitions), the done button 772 is pressed when a set of repetitions completed. In another implementation where weights are repeatedly lifted, the done button 772 is pressed after a predefined number of sets of repetitions has been completed. In such an implementation, individual sets are identified when a predefined minimum time delay threshold between consecutive repetitions has been satisfied. In another implementation, a user presses the start button at the beginning and then presses stop when done, regardless or repetitions or sets. In this case, the device displaying the graphical code will use an algorithm to deten-nine sets. For instance, a pause of more than 15 seconds becomes the start of a new set of repetitions. In some implementations, when the done button 772 is not depressed or otherwise actuated after predetermined time, controller 50 automatically deems an individual workout set or session to of been completed.

As shown by Figure 10, display 740, in response to signals from a fitness equipment unit 24 (or 524) representing values of one or more exercise metrics, and in response to control signals from controller 50 (shown in Figures 5 and 8), present a generated graphical code 54A. Once the graphical code 54A has been presented, portable electronic device 800 may be used to capture the presented graphical code 54A.

In the example illustrated, portable electronic device 800 comprises input 804, display 306 and code capture device 308, in addition to the other components shown and described above with respect to Figure 5. Input 804 comprise a push button that may utilize initiate the capture of graphical code 54A by code capture device 308, In one implementation, input 804 may be incorporated as part of a touchscreen with display 306. Code capture device 308 comprises a camera (the lens of which is shown). Although shown is a front positioned camera, code capture device 308 may alternatively be located on a backside of portable electronic device 800, opposite to and facing away from display 306. The example illustrated, upon capture of graphical code 54A, processing unit 316 causes the captured graphical code 54A to be presented upon display 306, confirming such capture. As noted above with respect to a method 400 in Figure 6, the captured graphical code 54A, representing values for one or more exercise metrics, may be translated, stored and used to adjust an overall exercise routine.

Although the use of a dynamically generated and displayed graphical code to output dynamically changing information or data in response to changes in the information, (whether such changes are the result of input by a person or sensed by a sensor) has been described with respect to a fitness equipment unit or exercise machine, in other implementations, the use of dynamically generated and displayed graphical codes to output dynamically changing information or data may be extended to other applications. For example, in other applications where it may be desirable to output dynamically changing information or data from a machine, device or environment for capture, use and possible transmission to a higher computing device for processing and storage, in those circumstances where such output is difficult due to insufficient power, processing or transmission capabilities of the machine, device or environment, the above-described output systems may be extended to such other machines, devices or environments. In terms of a specific example, such device or machine having limited power, processing or wireless or wired transmission capabilities may be provided with a low-power, low-cost, low processing device which simply presents a graphical code based on sensed changes or user input changes in settings or parameters, wherein the graphical code may be subsequently captured by a portable electronic device, allowing the data represented by the graphical code to be easily transmitted, translated, stored and processed by another device having sufficient power and processing capabilities.

Although the present disclosure has been described with reference to example embodiments, workers skilled in the art will recognize that changes may be made in form and detail without departing from the spirit and scope of the claimed subject matter. For example, although different example embodiments may have been described as including one or more features providing one or more benefits, it is contemplated that the described features may be interchanged with one another or alternatively be combined with one another in the described example embodiments or in other alternative embodiments. Because the technology of the present disclosure is relatively complex, not all changes in the technology are foreseeable. The present disclosure described with reference to the example embodiments and set forth in the following claims is manifestly intended to be as broad as possible. For example, unless specifically otherwise noted, the claims reciting a single particular element also encompass a plurality of such particular elements.

## Claims

1. An apparatus comprising:
a display; and
a controller to receive signals representing different values for an exercise metric of a fitness equipment unit and to generate control signals causing the display to present different graphical codes based on the different values for the exercise metric.

2. The apparatus of claim 1, comprising at least one sensor to sense the exercise metric of the fitness equipment unit, wherein a sensor optionally comprises a reed switch.

3. The apparatus of claim 1 or 2, wherein the display and the controller are powered by a battery, wherein the apparatus optionally comprises a photovoltaic cell to recharge the battery.

4. The apparatus of claim 1,2 or 3, wherein at least one graphical code comprises at least one of a quick response (QR) code and a barcode.

5. The apparatus of any preceding claim, when an exercise metric represented by at least one graphical code comprises at least one of motion of a movable member of the fitness equipment unit, a number of repetitions of movement of a movable member of the fitness equipment unit, a speed of movement of a movable member of the fitness equipment unit, a time duration of movement of a movable member of the fitness equipment unit, a level of resistance to movement of a movable member of the fitness equipment unit, and which of a plurality of selectable weights of the fitness equipment unit has been selected.

6. The apparatus of any preceding claim, wherein at least one graphical code indicates an identity of the fitness equipment unit.

7. The apparatus of any preceding claim, wherein at least one graphical code identifies a day on which exercise is performed.

8. The apparatus of any preceding claim, wherein at least one graphical code represents at least two exercise metrics for the fitness equipment unit.

9. The apparatus of any preceding claim, wherein the display and the controller are removably mounted to the fitness equipment unit.

10. The apparatus of any preceding claim, wherein the fitness equipment unit comprises a stack of selectable weights to be repeatedly moved by a person during exercise, and wherein an exercise metric represented by at least one graphical code comprises at least one of a selected weight of the stack of selectable weights, a number of times at which a selected weight has been lifted, and a distance at which the selectable weight has been lifted.

11. The apparatus of any preceding claim, comprising an input to indicate completion of an exercise, wherein the controller causes generation of at least one graphical code in response to the input indicating completion of the exercise.

12. The apparatus of any preceding claim, comprising:
a code capture device to capture a digital image of at least one graphical code; and
a non-transient computer-readable medium storing computer-readable code to translate the digital image of the at least one graphical code into at least one value for an exercise metric.

13. The apparatus of any preceding claim, comprising a portable electronic device including a code capture device and a non-transient computer-readable medium, and optionally comprising a remote computing device to receive signals from the portable electronic device representing at least one value for an exercise metric and to transmit at least one message for display by the portable electronic device based upon the at least one value for the exercise metric,

14. The apparatus of any preceding claim, comprising:
a portable electronic device comprising:
a portable electronic device and
a code reader,
wherein the apparatus optionally comprises a remote computing device to receive signals from the portable electronic device representing at least one value for an exercise metric and to transmit at least one message for display by the portable electronic device based upon the at least one value for the exercise metric.

15. A method comprising:
receiving signals representing different values for an exercise metric of a fitness equipment unit; and
generating and displaying different graphical codes based on different values received for the exercise metric.
